# EUROPEAN PATENT APPLICATION

(11) **EP 4 292 604 A1**
(43) Date of publication of application: **20.12.2023**
(21) Application number: 22739735.3
(22) Date of filing: 13.01.2022
(51) Int. Cl.: A61K 38/21, A61K 9/00, A61P 31/12, A61P 31/14

(54) **PROPHYLACTIC ADMINISTRATION METHOD AGAINST RESPIRATORY VIRUS, COMPRISING ADMINISTERING INTERFERON-BETA TO POTENTIAL RESPIRATORY VIRUS-INFECTED SUBJECT**

(30) Priority: 13.01.2021 KR 20210004780
(71) Applicant: Abion Inc., Seoul 08394 (KR)
(72) Inventor: CHOI, Jun Young, Seoul 08394 (KR); KIM, Na Young, Seoul 08394 (KR); SON, Won Rak, Seoul 08394 (KR); CHOI, Hong Seok, Seoul 08394 (KR); LEE, Young Jin, Seoul 08394 (KR); HONG, Sung Hyun, Seoul 08394 (KR)
(74) Representative: Schwarz & Partner Patentanwälte GmbH
(86) International application number: PCT/KR2022/000683
(87) International publication number: WO 2022/154542

(57) **Abstract**

The present invention relates to a prophylactic administration method against respiratory viruses, comprising administering interferon-beta to a potential respiratory virus-infected subject, and more specifically, to a method for restraining self-replication infection of respiratory viruses in a subject exposed to potential respiratory virus infection by administering interferon-beta as an active ingredient to respiratory cells through breathing in the manner of direct administration to cells infected with or potentially infected with a respiratory virus.

## Description

### TECHNICAL FIELD

This application claims the priority of Korean Patent Application No. 10-2021-0004780, filed on January 13, 2021, the entirety of which is a reference of the present application.

The present invention relates to a prophylactic administration method against respiratory viruses, comprising administering interferon-beta to a potential respiratory virus-infected subject, and more specifically, to a method for restraining self-replication infection of respiratory viruses in a subject exposed to potential respiratory virus infection by administering interferon-beta as an active ingredient to respiratory cells through breathing in the manner of direct administration to cells infected with or potentially infected with a respiratory virus.

### BACKGROUND ART

Respiratory diseases caused by respiratory infections of viruses are the most prevalent diseases, accounting for about half of all infectious diseases. The respiratory virus infections occur mainly in children, elderly, immunodepressed patients, and the most well-known respiratory infectious viruses include adenovirus, parainfluenza virus (PIV), respiratory syncytial virus (RSV), rhinovirus, coronavirus, and the like.

Among respiratory viruses, coronaviruses are divided into four genera, alpha and beta genera infect humans and animals, and gamma and delta genera infect animals only. There are six known types of coronavirus that can infect humans. Among them, four types 229E, OC43, NL63, and HKU1 are known as viruses that cause colds, and the rest of two types are viruses that can cause severe pneumonia in humans: middle east respiratory syndrome coronavirus (MERS-CoV) and severe acute respiratory syndrome coronavirus (SARS-CoV).

In particular, a novel respiratory virus first identified in a pneumonia patient in Wuhan City, Hubei Province, China in December 2019 severely affected a medical system due to a wide spread. The novel coronavirus occurring in 2019 was identified as a different family from the existing MERS-CoV and SARS-CoV viruses, and was identified as the seventh coronavirus species infecting humans. The corresponding virus is named 'SARS-CoV-2' and causes a disease called 'Coronavirus Disease 2019 (COVID-19)'.

Since initial symptoms of respiratory virus infection show similar symptoms and signs regardless of viruses, it is difficult to differentiate a cause based only on clinical symptoms, and since the virus is highly contagious, the virus may also cause a pandemic in a short time.

### [Prior Arts]

### [Patent Documents]

(Patent Document 1) Korean Patent Registration No. 10-2018201
(Patent Document 2) Korean Patent Registration No. 10-1800366
(Patent Document 3) Korean Patent Publication No. 10-2019-0063512

### [Non-Patent Document]

(Non-Patent Document 1) Zhu, Na, et al. "A novel coronavirus from patients with pneumonia in China, 2019." New England Journal of Medicine (2020).

### DISCLOSURE

### TECHNICAL PROBLEM

Therefore, the present inventors have studied interferon-beta to be used to restrain respiratory virus infection, confirmed that the interferon-beta may restrain self-replication infection of respiratory viruses in subjects at risk of exposure to respiratory virus infection, that is, subjects exposed to potential respiratory virus infections when properly administered to a respiratory tract region where infection of respiratory viruses usually occurred, and then completed the present invention.

An object of the present invention is to provide a pharmaceutical composition for restraining self-replication infection of respiratory viruses in a subject exposed to potential respiratory virus infection including interferon-beta as an active ingredient.

In addition, an object of the present invention is to provide a pharmaceutical composition for restraining self-replication infection of respiratory viruses in a subject exposed to potential respiratory virus infection consisting of interferon-beta.

In addition, an object of the present invention is to provide a pharmaceutical composition for restraining self-replication infection of respiratory viruses in a subject exposed to potential respiratory virus infection essentially consisting of interferon-beta.

Another object of the present invention is to provide a post-exposure prophylaxis (PEP) method for protecting a subject from self-replication infection by respiratory viruses after exposed to potential respiratory virus infection, in which a prophylactically effective amount of interferon-beta is administered to a subject.

Yet another object of the present invention is to provide a method for protecting a subject from self-replication infection by respiratory viruses including administering a prophylactically effective amount of interferon-beta to a subject after exposed to potential respiratory virus infection.

Still another object of the present invention is to provide a use of interferon-beta for preparing an agent for restraining self-replication infection of respiratory viruses in a subject exposed to potential respiratory virus infection.
Still yet another object of the present invention is to provide a method for restraining self-replication infection of respiratory viruses in a subject exposed to potential respiratory virus infection including administering an effective amount of a composition including interferon-beta as an active ingredient to a subject in need thereof.

### TECHNICAL SOLUTION

In order to achieve the object of the present invention, the present invention provides a pharmaceutical composition for restraining self-replication infection of respiratory viruses in a subject exposed to potential respiratory virus infection including interferon-beta as an active ingredient.

Further, the present invention provides a pharmaceutical composition for restraining self-replication infection of respiratory viruses in a subject exposed to potential respiratory virus infection consisting of interferon-beta.

Further, the present invention provides a pharmaceutical composition for restraining self-replication infection of respiratory viruses in a subject exposed to potential respiratory virus infection essentially consisting of interferon-beta.

In order to achieve another object of the present invention, the present invention provides a post-exposure prophylaxis (PEP) method for protecting a subject from self-replication infection by respiratory viruses after exposed to potential respiratory virus infection, in which a prophylactically effective amount of interferon-beta is administered to a subject.

In order to achieve yet another object of the present invention, the present invention provides a method for protecting a subject from self-replication infection by respiratory viruses including administering a prophylactically effective amount of interferon-beta to a subject after exposed to potential respiratory virus infection.

In order to achieve still another object of the present invention, the present invention provides a use of interferon-beta for preparing an agent for restraining self-replication infection of respiratory viruses in a subject exposed to potential respiratory virus infection.

In order to achieve still yet another object of the present invention, the present invention provides a method for restraining self-replication infection of respiratory viruses in a subject exposed to potential respiratory virus infection including administering an effective amount of a composition including interferon-beta as an active ingredient to a subject in need thereof.

Unless otherwise defined, all technical and scientific terms used herein have the same meaning as commonly understood by those skilled in the art. The following references provide one skill with general definitions of various terms used in the present specification: Singleton et al., DICTIONARY OF MICROBIOLOGY AND MOLECULAR BIOLOTY(2th ed. 1994); THE CAMBRIDGE DICTIONARY OF SCIENCE AND TECHNOLOGY(Walkered., 1988); and Hale & Marham, THE HARPER COLLINS DICTIONARY OF BIOLOGY.

Hereinafter, the present invention will be described in detail.

The present invention provides a pharmaceutical composition for restraining self-replication infection of respiratory viruses in a subject exposed to potential respiratory virus infection including interferon-beta as an active ingredient.

Interferon-beta is a globular protein having 5 alpha helices and a size of 22 kDa. In addition, the interferon-beta has various immunological activities, such as antiviral activity, cell growth inhibition or antiproliferative activity, lymphocyte cytotoxicity enhancing activity, immunomodulatory activity, differentiation induction or inhibitory activity of target cells, macrophage activation activity, increased activity of cytokine production, increased activity of cytotoxic T cell effect, increased activity of natural killing cells, etc. Accordingly, it has been reported that the interferon-beta is effective in treating cancer, autoimmune disorders, viral infections, HIV-related diseases, hepatitis C, rheumatoid arthritis, and the like.

The interferon-beta of the present invention may be natural type (wild type) or mutant type interferon-beta, and preferably, the interferon-beta (interferon-beta polypeptide or interferon-beta protein) of the present invention may be a polypeptide consisting of an amino acid sequence represented by SEQ ID NO: 1, in which an arginine 27 (R27) site of the wild-type interferon-beta is mutated to threonine (R27T). The interferon-beta of the present invention should be understood as a polypeptide having interferon-beta activity, in which a sugar chain binds to two sites of amino acids at positions 25 and 80 of SEQ ID NO: 1. Preferably, the interferon-beta of the present invention refers to human interferon-beta.

In the present invention, the interferon-beta is administered by directly reaching cells infected with or potentially infected with coronavirus. More specifically, the interferon-beta comes into contact with cells infected with or potentially infected with respiratory viruses by inhalation administration, and has a function by reaching cells infected with or potentially infected with respiratory viruses to be bound with an interferon beta receptor of the cells.

The inhalation administration is performed through the oral cavity or nasal cavity due to the structure of a respiratory system, and for example, interferon-beta or a carrier containing interferon-beta may be sprayed or injected to be in contact with respiratory cells through the oral cavity or nasal cavity in a liquid, aerosol or gaseous state.

In the present invention, the respiratory system refers to an organ in which breathing is performed as a whole, and refers to each organ starting from the nose and mouth and leading to the airways and lungs. Preferably, in the present invention, the respiratory system includes nasal mucosa, nasopharynx, oropharynx, laryngopharynx, larynx, trachea, bronchi, bronchioles, and lungs.

In the present invention, the respiratory virus may be selected from the group consisting of Adenovirus, Avian Influenza Virus, Bocavirus, Coronavirus, Cytomegalovirus, Hantavirus, Herpes Simplex Virus, Influenza Virus, Measles, Metapneumovirus, Parainfluenza Virus, Respiratory Syncytial Virus, Rhinovirus, and Varicella-zoster Virus. Preferably, the respiratory virus of the present invention is coronavirus.

In the present invention, the subject may be a subject who is not infected with coronavirus or who is in the early stage of coronavirus infection. The subject may be a subject at risk of exposure to coronavirus, either clinically or regulatoryly (for example, a subject close contact with a coronavirus patient or a subject contact with a close contact subject, a subject to be advised or enforced in quarantine, and a healthcare worker engaged in coronavirus treatment), an asymptomatic carrier infected with coronavirus, and an initial infected subject of coronavirus, and the subject infected with coronavirus may be easily identified by those skilled in the art using known coronavirus diagnostic kits. Preferably, the subject of the present invention may be a person who is not infected with coronavirus or who is in the early stage of coronavirus infection. Therefore, the present invention may restrain or reduce the spread of the virus to the surroundings while a subject infected with coronavirus is in an asymptomatic state.

These pre-exposure prophylaxis (PrEP) and post-exposure prophylaxis (PEP) may be applied similarly to a method for human immunodeficiency virus (HIV) prophylaxis.

In the present invention, the subject in the early stage of coronavirus infection may be a subject who has no coronavirus infection symptom selected from the group consisting of fever, dry cough, fatigue, body aches, sore throat, diarrhea, conjunctivitis, headache, loss of taste or smell, skin rash, and discoloration of fingers or toes.

In the present invention, the coronavirus may be any one of i) alphacoronavirus such as human-infected 229E and NL63 or human-uninfected porcine epidemic diarrhea virus (PEDV), (porcine) transmissible gastroenteritis virus (TGEV), canine coronavirus (CCoV), feline coronavirus (FCoV), Miniopterus bat coronavirus 1, Miniopterus bat coronavirus HKU8, Rhinolophus bat coronavirus HKU2, and Scotophilus bat coronavirus 512; ii) betacoronavirus such as human-infected OC43, HKU1, SARS-CoV, MERS-CoV, and SARS-CoV-2 or human-uninfected porcine hemagglutinating encephalomyelitis virus (PHEV), bovine coronavirus (BCoV), equine coronavirus (EqCoV), murine coronavirus (MuCoV), Tylonycteris bat coronavirus HKU4, Pipistrellus bat coronavirus HKU5, and Rousettus bat coronavirus HKU9; iii) gammacoronavirus such as human-uninfected Avian coronavirus and Beluga whale-coronavirus SW1; and iv) deltacoronavirus such as human-uninfected Bulbul-coronavirus HKU11, Thrush-coronavirus HKU12, and Munia-coronavirus HKU13.

In the present invention, the self-replication infection indicates that the coronavirus replicates in the respiratory cells of the subject and infects surrounding cells. The life cycle of coronavirus is well known.

Meanwhile, the present invention provides a post-exposure prophylaxis (PEP) method for protecting a subject from self-replication infection by respiratory viruses after exposed to potential respiratory virus infection, in which a prophylactically effective amount of interferon-beta is administered to a subject.

Further, the present invention provides a method for protecting a subject from self-replication infection by respiratory viruses including administering a prophylactically effective amount of interferon-beta to a subject after exposed to potential respiratory virus infection.

Further, the present invention provides a method for protecting a subject from self-replication infection caused by coronavirus, further including concomitantly or sequentially administering a clinical-ongoing or known coronavirus therapeutic agent.

The administration of the coronavirus therapeutic agent may be oral, inhalational, intraperitoneal or intravenous administration.

The pharmaceutical composition according to the present invention may contain the coronavirus therapeutic agent of the present invention alone or may further contain one or more pharmaceutically acceptable carriers, excipients or diluents.

The pharmaceutically acceptable carrier may further include, for example, a carrier for oral administration or a carrier for parenteral administration. The carrier for oral administration may include lactose, starch, cellulose derivatives, magnesium stearate, stearic acid, and the like. In addition, the carrier for parenteral administration may include water, suitable oil, saline, aqueous glucose, glycol, and the like, and further include a stabilizer and a preservative. A suitable stabilizer includes antioxidants such as sodium hydrogen sulfite, sodium sulfite or ascorbic acid. A suitable preservative includes benzalkonium chloride, methyl- or propyl-paraben and chlorobutanol. Other pharmaceutically acceptable carriers may refer to those known in the art.

The composition of the present invention preferably includes an active ingredient and a pharmaceutically acceptable carrier in a weight ratio of 0.1 to 99.9: 99.9 to 0.1, but is not limited thereto.

The pharmaceutical composition of the present invention may be administered to mammals including humans even by any method. For example, the pharmaceutical composition of the present invention may be administered orally or parenterally, and may be formulated into a preparation for oral administration or parenteral administration depending on a route of administration.

In the case of the formulations for oral administration, the composition of the present invention may be formulated as powders, granules, tablets, pills, sugar-coated tablets, capsules, liquids, gels, syrups, slurry, suspensions, etc. by using methods known in the art. For example, the oral formulation may obtain tablets or sugar-coated tablets by mixing an active ingredient with a solid excipient, pulverizing the mixture, adding a suitable adjuvant, and then processing the mixture into a granular mixture. Examples of the suitable excipient may include sugars including lactose, dextrose, sucrose, sorbitol, mannitol, xylitol, erythritol and maltitol, starches including corn starch, wheat starch, rice starch and potato starch, celluloses including cellulose, methyl cellulose, sodium carboxymethylcellulose and hydroxypropylmethyl-cellulose, fillers such as gelatin, polyvinylpyrrolidone, and the like. In addition, in some cases, cross-linked polyvinylpyrrolidone, agar, alginic acid, sodium alginate, or the like may be added as a disintegrant. In addition, the pharmaceutical composition of the present invention may further include anti-coagulating agents, lubricants, wetting agents, flavorings, emulsifiers, antiseptics, etc.

The formulations for parenteral administration may be formulated in the form of injections, creams, lotions, external ointments, oils, moisturizers, gels, aerosols and nasal inhalants by methods known in the art. These formulations are described in formulary commonly known in all pharmaceutical chemistry.

The total effective dose of the composition of the present invention may be administered to a patient in a single dose, or may be administered in a multiple dose for a long period of time according to a fractionated treatment protocol. In the pharmaceutical composition of the present invention, the content of the active ingredient may vary depending on the severity of disease. Preferably, the preferred total dose of the pharmaceutical composition of the present invention may be about 0.01 to 10,000 mg, most preferably 0.1 to 500 mg per 1 kg of patient's body weight per day. However, in the dose of the pharmaceutical composition, since the effective dose to the patient is determined by considering various factors including the age, body weight, health conditions, and sex of a patient, the severity of a disease, diet, and excretion rate as well as a formulation method, a route of administration and the number of treatment times, those skilled in the art may determine a suitable effective dose of the composition of the present invention by considering these factors. The pharmaceutical composition according to the present invention is not particularly limited to the formulations, the administration routes, and the administration methods thereof, as long as the effects of the present invention are shown.

The present invention provides a use of interferon-beta for preparing an agent for restraining self-replication infection of respiratory viruses in a subject exposed to potential respiratory virus infection.

The present invention provides a method for restraining self-replication infection of respiratory viruses in a subject exposed to potential respiratory virus infection including administering an effective amount of a composition including interferon-beta as an active ingredient to a subject in need thereof.

The 'effective dose' of the present invention means an amount that exhibits effects of improving, treating, preventing, detecting, and diagnosing respiratory virus infection, or restraining or alleviating the disease when administered to the subject. The 'subject' may be animals, preferably, mammals, particularly animals including humans, and may also be cells, tissues, organs, and the like derived from animals. The subject may be a patient requiring the effects.

The term "comprising" used herein is used in the same meaning as "including" or "characterized by", and does not exclude additional ingredients or steps of the method, which are not specifically mentioned in the composition or the method according to the present invention. In addition, the term "consisting of" means excluding additional elements, steps or ingredients, etc., unless otherwise described. The term "essentially consisting of" means including materials or steps which do not substantially affect basic properties thereof in addition to the described materials or steps within the range of the composition or the method.

### [Advantageous Effects]

As described above, according to the present invention, it is possible to effectively restrain self-replication infection of coronavirus in a subject exposed to potential coronavirus infection by administering interferon-beta by breathing of a respiratory system such as the nasal mucosa, nasopharynx, oropharynx, laryngopharynx, larynx, trachea, bronchi, bronchioles, lungs, etc.

### DESCRIPTION OF DRAWINGS

FIGS. 1a and 1b illustrate results of confirming an effect of restraining coronavirus infection by interferon by treating cells with interferon beta before (FIG. 1a) or after (FIG. 1b) infection with SARS-CoV-2.
FIGS. 2a and 2b illustrate results of quantitatively analyzing the results of FIGS. 1a and 1b.
FIG. 3 illustrates results of confirming an effect of restraining coronavirus infection by Remdesivir and interferon beta by a plaque assay (left) and real-time quantitative RT-qPCR (right).
FIG. 4 illustrates a result of confirming an effect of restraining lung damage caused by coronavirus infection by interferon-beta in a hamster infection model.
FIG. 5 illustrates a schematic experiment sequence for analyzing a pre-exposure prophylaxis effect of interferon-beta against a coronavirus Wuhan strain or delta variant.
FIG. 6 illustrates a result of confirming a pre-exposure prophylaxis effect of interferon-beta against a coronavirus Wuhan strain or delta variant by a plague assay.
FIG. 7 illustrates a schematic experiment sequence for analyzing a post-exposure prophylaxis or therapeutic effect of interferon-beta against a coronavirus Wuhan strain or delta variant.
FIG. 8 illustrates a result of confirming a post-exposure prophylaxis or therapeutic effect of interferon-beta against a coronavirus Wuhan strain or delta variant by a plague assay.
FIG. 9 illustrates a schematic experiment sequence for analyzing a prophylactic effect of interferon-beta against influenza (H1N1).
FIG. 10 illustrates a result of confirming a prophylactic effect of interferon-beta against influenza (H1N1) by a plague assay.

### MODES FOR THE INVENTION

Hereinafter, preferred embodiments will be proposed in order to help in understanding of the present invention. However, the following Examples are just provided to more easily understand the present invention and the contents of the present invention are not limited by Examples.

### Example 1: Coronavirus infection restraint of interferon-beta

For a coronavirus infection restraint experiment of interferon-beta, RT-qPCR was performed by extracting RNA after infecting Vero E6 cells and treating interferon-beta. Treatment conditions were as follows:
Vero cells were grown in DMEM media containing 2% heat-inactivated FBS and 2 mM L-glutamine, and seeded in a 6-well at 5 × 10⁵ cells/well and incubated in a 37°C, 5% CO₂ incubator to maintain about 80% confluence. The next day after seeding, in a pre-infection experiment, an SARS-CoV-2 stock was first infected with about 2 × 10⁶ plaque-forming units (PFU/mL), and then treated with an ABN101 substance, and in the case of post-infection, the SARS-CoV-2 stock was first treated with the ABN101 substance by concentration and then infected and experimented.

In the pre-infection experiment, Vero E6 cells were treated with SARS-CoV-2 for 1 hour and 30 minutes and infected, and then added with 3 ml of culture media after the infected viruses were completely removed, and then diluted and treated with interferon-beta (ABN101, R27T interferon-beta) to 5000 IU/mL, 1,000 IU/mL, 2,000 IU/mL, and 5,000 IU/mL for each concentration. The cells were incubated for 3 days in a 37°C, 5% CO₂ incubator, and then each culture supernatant was recovered. Each supernatant was filtered to remove the cells, and total RNA was isolated. It was confirmed whether the SARS-CoV-2 virus has been reduced by treating interferon-beta through quantitative real-time RT-qPCR by using the isolated RNA as a template and a primer capable of specifically detecting an E gene of SARS-CoV-2. Primer and probe sequences for detecting the E gene of SARS-CoV-2 used in the experiment were as follows.
Probe sequence: 5'-ATATTGCAGCAGTACGCACACA-3' (SEQ ID NO: 2)
Primer-1 sequence: 5'-ACAGGTACGTTAATAGTTAATAGCGT-3' (SEQ ID NO: 3)
Primer-2 sequence: 5'-ATATTGCAGCAGTACGCACACA-3' (SEQ ID NO: 4)

In the post-infection experiment, interferon-beta (ABN101, R27T interferon-beta) was diluted to 5000 IU/mL, 1,000 IU/mL, 2,000 IU/mL, and 5,000 IU/mL for each concentration and treated for 24 hours to remove the culture media, and Vero E6 cells were treated with SARS-CoV-2 for 1 hour and 30 minutes to infect the cells, and then the infected virus was completely removed, and then 3 ml of culture media were added. The cells were incubated in a 37°C, 5% CO₂ incubator, and then on the second day, the culture supernatant was collected and filtered to remove the cells, and total RNA was isolated. A RT-qPCR experiment was performed in the same manner as the pre-infection experiment.

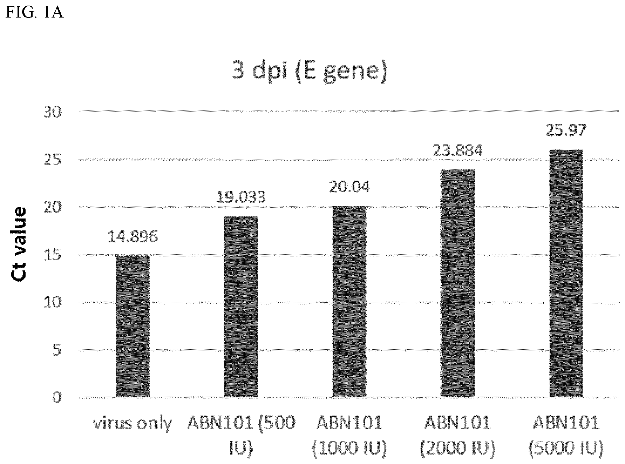

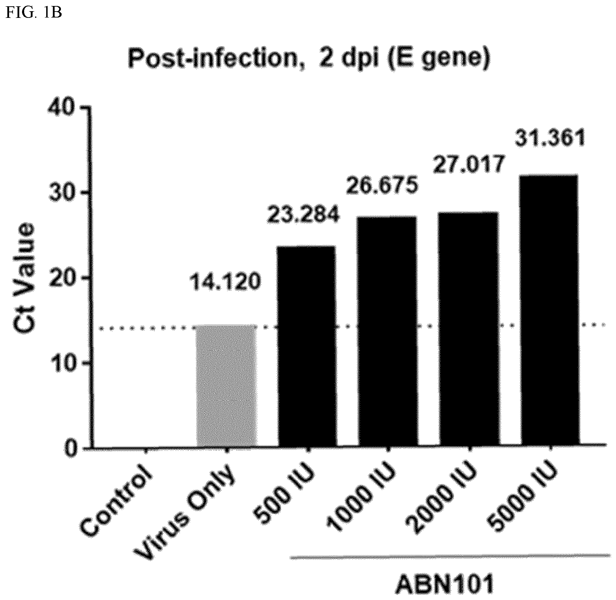

As a result, as illustrated in FIG. 1, in both pre-infection and post-infection, when only virus was treated (virus only), a Ct value was 14.896, whereas in the case of treating interferon-beta at 500 IU, 1000 IU, 2000 IU, and 5000 IU (ABN101 (treatment amount)), as the treatment amount of interferon-beta increased, the Ct value increased, and thus it could be seen that the amount of virus decreased in a concentrationdependent manner.

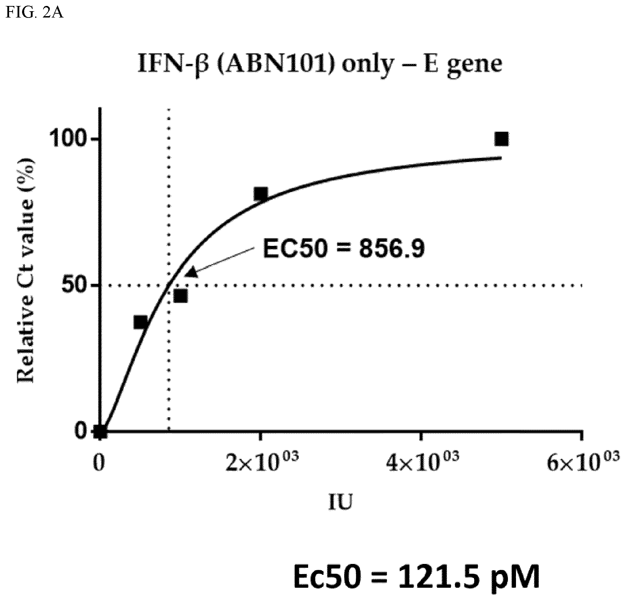

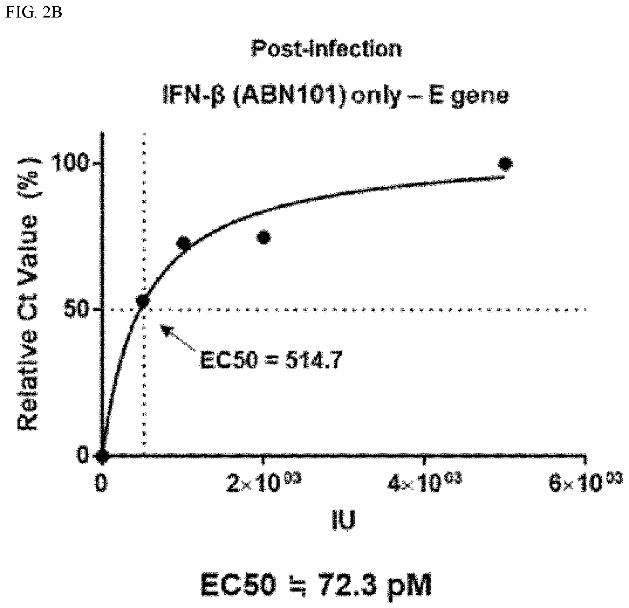

The quantitative analysis was illustrated in FIG. 2, and it was confirmed that the EC50 of ABN101 against SARS-CoV-2 was 121.5 pM (pre-infection) and 72.3 pM (post-infection), respectively.

### Example 2: Comparison of coronavirus infection restraint effect with Remdesivir

To compare a coronavirus infection restraint effect of interferon-beta with that of Remdesivir, the effects were compared through a plaque assay and real-time quantitative RT-qPCR, respectively.

In the plaque assay, after overlaying the cells with agarose, the virus plaques were analyzed by staining with crystal violet to determine how much the cells were infected with the virus.

Vero cells were grown in DMEM media containing 2% heat-inactivated FBS and 2 mM L-glutamine, and seeded in a 6-well at 5 × 10⁵ cells/well and incubated in a 37°C, 5% CO₂ incubator to maintain about 80% confluence. The next day after seeding, a SARS-CoV-2 stock was treated with about 2 × 10⁶ plaque-forming units (PFU/mL).

Vero E6 cells were treated with SARS-CoV-2 for 1 hour and 30 minutes to be infected, and then the infected viruses were completely removed, and then 3 mL of the culture media were added, and then Remdesivir was treated by concentration and incubated in a 37°C, 5% CO₂ incubator for 3 days, and then the culture supernatant was recovered.

Remdesivir was dissolved in DMSO and prepared, and Mock-1 and Mock-2 were prepared by setting a control a substances containing DMSO as a measurement standard.

Real-time quantitative RT-qPCR was performed by treating Remdesivir at 0.01 µM (10 nM), 0.05 µM (50 nM), 0.1 µM (100 nM), 0.2 µM (200 nM), 0.5 µM, 1 µM, 2 µM, and 3 µM, respectively, and the experiment was conducted as in Example 1.

Treatment substances for Mock-1 and Mock-2 were as follows.
Mock-1: culture media only after virus infection
Mock-2: culture media + DMSO after virus infection

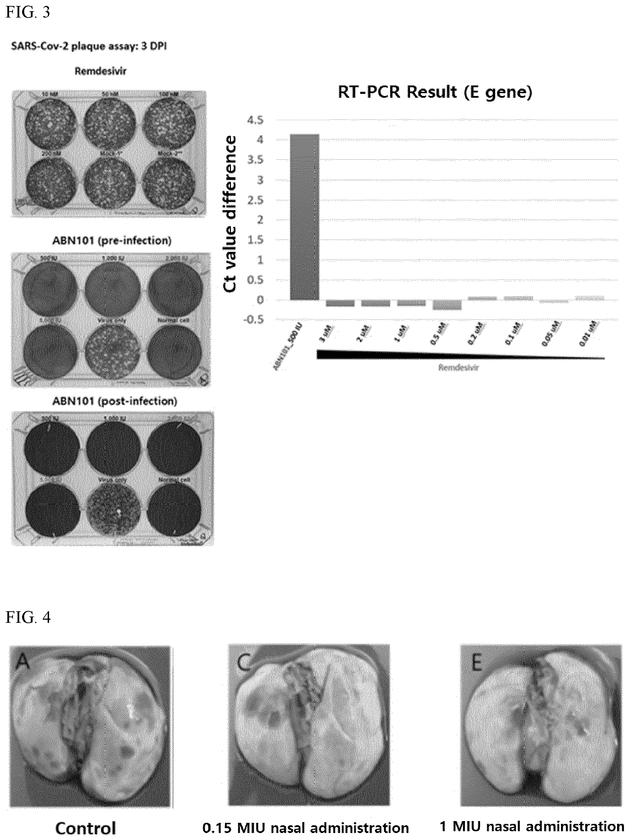

The left side of FIG. 3 illustrates a result of the plaque assay, and it could be seen that in the case of pre-infection and post-infection treatment with interferon-beta (bottom left), respectively, infected cells were not identified so much, whereas in the case of Remdesivir treatment (upper left), viral infection was not significantly restrained. The EC50 of Remdesivir was known as 74 nM, but it was confirmed that the EC50 of interferon-beta showed good efficacy at a concentration about 600 times lower therethan.

The right side of FIG. 3 was a result of real-time quantitative RT-qPCR, and the experiment was conducted in the same manner as in FIG. 1. As a result, there was little difference in Ct value between Remdesivir and virus when only virus was treated (virus only), whereas when 500 IU of interferon-beta was treated (ABN101 (treatment amount)), a difference in Ct value between interferon-beta and virus only increased, and thus, it was confirmed that the efficacy of interferon-beta was superior to the EC50 of Remdesivir.

### Example 3: Effect of restraining lung damage caused by coronavirus infection by interferon-beta in hamster infection model

Since it was a well-known fact that lung damage was caused by infection with SARS-CoV-2, it was confirmed whether interferon-beta restrained lung damage caused by coronavirus infection.

In order to confirm the degree of lung damage by infection with SARS-CoV-2, 6-week-old male Syrian Hamster was prepared and infected with a virus at 100 PFU/head by nasal administration. Immediately after infection (0 day post-injection (d.p.i.)), 2 days later (2 d.p.i), and 4 days later (4 d.p.i), interferon-beta (ABN101) was administered total three times at 0.15 MIU/head and 1 MIU/head, respectively, and then lung tissue damage was evaluated.

In a control group, virus only was administered (control, virus only), and in an experimental group, interferon-beta was administered to a respiratory system in amounts of 0.15 MIU (million IU) and 1 MIU through nasal administration, respectively. The degree of lung damage was evaluated by a third-party expert with a veterinary license, and after sacrificing the hamster, the entire lung was extracted, and the lungs of the virus-only group and the interferon-beta-administered group were numbered and blinded to measure the evaluated damage degree. This was evaluated by a lung damage scoring scale ranging from 0 to 10 points, the entire lung area damage was measured to 10 points, and a normal level was measured to 0 point.

As a result, as illustrated in FIG. 4 and Table 1, it was confirmed that when only the virus was administered, the lung damage score was 3.8 ± 0.98, whereas when interferon-beta was administered, the lung damage score was reduced in a dosedependent manner.

**Table 1**

| Group | Virus administration (PFU/head) | Administration time | Lung damage score |
|---|---|---|---|
| Virus only | 100 | - | 3.8 ± 0.98 |
| ABN101 0.15 MIU Nasal administration | 100 | 0, 2, 4 DPI | 3.5 ± 1.69 |
| ABN101 1 MIU Nasal administration | 100 | 0, 2, 4 DPI | 3.1 ± 1.63 |

### Example 4: Prophylactic effect of interferon-beta against coronavirus Wuhan strain or delta variant

To experiment a coronavirus infection prophylactic (pre-exposure prophylaxis) effect of interferon-beta, Vero E6 cells were treated with interferon-beta, then removed, and Wuhan or delta variant coronavirus was infected and analyzed by a plaque assay.

In the plaque assay, after overlaying the cells with agarose, the virus plaques were analyzed by staining with crystal violet to determine how much the cells were infected with the virus.

Vero E6 cells were grown in Complete DMEM media (10% FBS), and the Vero E6 cells were seeded in 12-well and incubated in a 37°C, 5% CO₂ incubator to maintain about 80% confluence. A SARS-CoV-2 stock (Wuhan strain or delta variant) was treated with about 100 plaque-forming units (PFU/mL) to measure a prophylactic effect.

To confirm prophylactic activity, ABN101 was treated at 25 IU/mL, 50 IU/mL, 100 IU/mL, 250 IU/mL, 500 IU/mL, 1,000 IU/mL, 2,500 IU/mL, 5,000 IU/mL, 10,000 IU/mL, and 25,000 IU/mL by concentration, and after 24 hours, ABN101 was removed. Thereafter, Vero E6 cells were treated with a Wuhan strain or delta variant of SARS-CoV-2 for 1 hour to infect the cells, and then after the infected virus was completely removed, the cells were incubated in new culture media for 72 hours, and a plaque assay was performed.

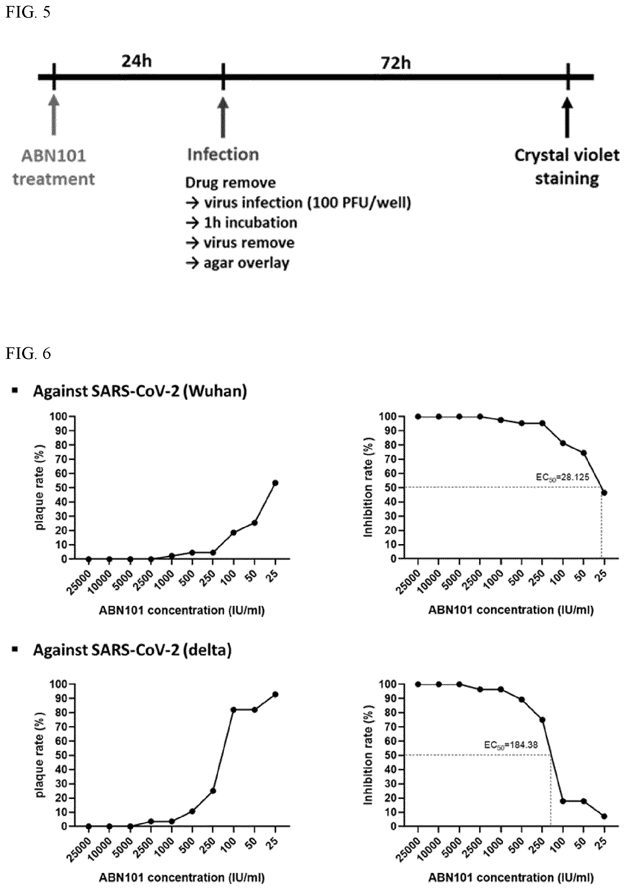

The procedure of the experiment was illustrated in FIG. 5.

As a result, as shown in Table 2 and FIG. 6, two coronavirus strains exhibited an infection prophylactic effect of 90% or higher at 250 IU/mL and an infection prophylactic effect of 95% or higher at 500 IU/mL or more, and the EC50 activity of ABN101 against the Wuhan strain was 28.125 IU/mL, and the EC50 activity against the Delta strain was 184.38 IU/mL.

**Table 2**

| **SARS-CoV-2 (Wuhan)** | | **SARS-CoV-2 (Delta)** | |
|---|---|---|---|
| **Concentration (IU/mL)** | **Plaque No.** | **Concentration (IU/mL)** | **Plaque No.** |
| 25000 | 0 | 25000 | 0 |
| 10000 | 0 | 10000 | 0 |
| 5000 | 0 | 5000 | 0 |
| 2500 | 0 | 2500 | 1 |
| 1000 | 1 | 1000 | 1 |
| 500 | 2 | 500 | 3 |
| 250 | 2 | 250 | 7 |
| 100 | 8 | 100 | 23 |
| 50 | 11 | 50 | 23 |
| 25 | 23 | 25 | 26 |
| 0 | 43 | 0 | 28 |
| Non-Infection | 0 | Non-Infection | 0 |

### Example 5: Post-exposure prophylaxis or therapeutic effect of interferon-beta against coronavirus Wuhan strain or delta variant

In order to experiment a coronavirus infection therapeutic effect of interferon-beta, Vero E6 cells were infected with Wuhan or delta variant coronavirus and then treated with interferon-beta by concentration and analyzed by a plaque assay.

In the plaque assay, after overlaying the cells with agarose, the virus plaques were analyzed by staining with crystal violet to determine how much the cells were infected with the virus.

The Vero E6 cells were grown in Complete DMEM media (10% FBS), and the Vero E6 cells were seeded in 12-well and incubated in a 37°C, 5% CO₂ incubator to maintain about 80% confluence. A SARS-CoV-2 stock (Wuhan strain or delta variant) was treated with about 100 plaque-forming units (PFU/mL) to measure a therapeutic effect.

To confirm efficacy, Vero E6 cells were treated with the Wuhan strain or delta variant of SARS-CoV-2 for 1 hour to infect the cells, and then the infected virus was completely removed, and ABN101 was treated for 72 hours at 25 IU/mL, 50 IU/mL, 100 IU/mL, 250 IU/mL, 500 IU/mL, 1,000 IU/mL, 2,500 IU/mL, 5,000 IU/mL, 10,000 IU/mL and 25,000 IU/mL by concentration, and the media were recovered, and then virus titration was measured to confirm infection restraint efficacy.

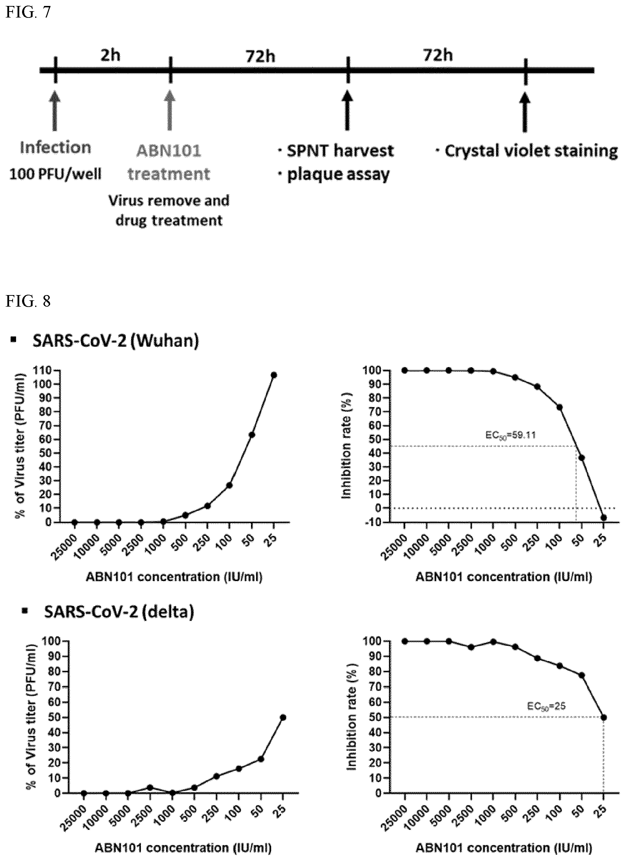

The flowchart of the experiment was illustrated in FIG. 7.

As a result, as shown in Table 3 and FIG. 8, the two coronavirus strains had the infection restraint efficacy of 95% or higher at 500 IU/mL or more. In the infection restraint efficacy of ABN101, the EC50 activity against the Wuhan strain was 59.11 IU/mL and the EC50 activity against the Delta strain was 25 IU/mL.

**Table 3**

| **SARS-CoV-2 (Wuhan)** | | **SARS-CoV-2 (Delta)** | |
|---|---|---|---|
| **Concentration (IU/mL)** | **Titration (PFU/mL)** | **Concentration (IU/mL)** | **Titration (PFU/mL)** |
| 25000 | 0.00 | 25000 | 1.00 x 10² |
| 10000 | 0.00 | 10000 | 1.10 x 10² |
| 5000 | 0.00 | 5000 | 1.80 x 10² |
| 2500 | 3.70 x 10² | 2500 | 3.10 x 10⁴ |
| 1000 | 1.80 x 10⁴ | 1000 | 3.00 x 10³ |
| 500 | 3.50 x 10⁵ | 500 | 3.00 x 10⁴ |
| 250 | 8.00 x 10⁵ | 250 | 9.00 x 10⁴ |
| 100 | 1.90 x 10⁶ | 100 | 1.30 x 10⁵ |
| 50 | 3.20 x 10⁶ | 50 | 1.80 x 10⁵ |
| 25 | 3.00 x 10⁶ | 25 | 4.00 x 10⁵ |
| 0 | 6.00 x 10⁵ | 0 | 8.00 x 10⁵ |

### Example 6: Influenza (H1N1) prophylactic effect of interferon-beta

To experiment an influenza infection prophylactic effect of interferon-beta, Vero E6 cells were treated with interferon-beta, then removed, and Influenza virus (H1N1/A/PR8) was infected and analyzed by a plaque assay.

In the plaque assay, after overlaying the cells with agarose, the virus plaques were analyzed by staining with crystal violet to determine how much the cells were infected with the virus.

MDCK cells were grown in Complete DMEM media (10% FBS), and the MDCK cells were seeded in 12-well and incubated in a 37°C, 5% CO₂ incubator to maintain about 80% confluence. An influenza virus stock (H1N1/A/PR8) was treated with about 100 plaque-forming units (PFU/mL) to measure a prophylactic effect.

To confirm prophylactic activity, ABN101 was treated at 25 IU/mL, 50 IU/mL, 100 IU/mL, 250 IU/mL, 500 IU/mL, 1,000 IU/mL, 2,500 IU/mL, 5,000 IU/mL, 10,000 IU/mL, and 25,000 IU/mL by concentration, and after 24 hours, ABN101 was removed. Thereafter, MDCK cells were treated with Influenza virus (H1N1/A/PR8) for 1 hour to infect the cells, and then after the infected virus was completely removed, the cells were incubated in new culture media for 72 hours, and a plaque assay was performed.

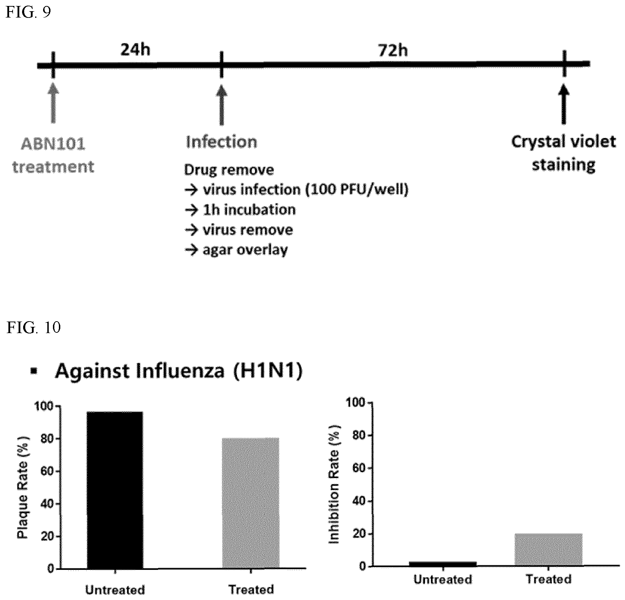

The sequence of the experiment was illustrated in FIG. 9.

As a result, as can be seen in FIG. 10, an ABN101 treated group showed an influenza infection prophylactic effect of 20% or more compared to an untreated group.

### INDUSTRIAL APPLICABILITY

As described above, according to the present invention, it is possible to effectively restrain self-replication infection of coronavirus in a subject exposed to potential coronavirus infection by administering interferon-beta by breathing of a respiratory system such as the nasal mucosa, nasopharynx, oropharynx, laryngopharynx, larynx, trachea, bronchi, bronchioles, lungs, etc.

## Claims

1. A pharmaceutical composition for restraining self-replication infection of respiratory viruses in a subject exposed to potential respiratory virus infection comprising interferon-beta as an active ingredient.

2. The pharmaceutical composition of claim 1, wherein the interferon-beta has an amino acid sequence of SEQ ID NO: 1.

3. The pharmaceutical composition of claim 1, wherein the interferon-beta is administered by inhalation.

4. The pharmaceutical composition of claim 3, wherein the administration by inhalation is performed in contact with respiratory cells by inhalation.

5. The pharmaceutical composition of claim 4, wherein the respiratory system includes nasal mucosa, nasopharynx, oropharynx, laryngopharynx, larynx, trachea, bronchi, bronchioles, and lungs.

6. The pharmaceutical composition of claim 1, wherein the respiratory virus is coronavirus, and the subject is a subject who is not infected with coronavirus or in the early stage of coronavirus infection.

7. The pharmaceutical composition of claim 6, wherein the subject in the early stage of coronavirus infection is a subject who has no coronavirus infection symptom selected from the group consisting of fever, dry cough, fatigue, body aches, sore throat, diarrhea, conjunctivitis, headache, loss of taste or smell, skin rash, and discoloration of fingers or toes.

8. The pharmaceutical composition of claim 1, wherein the respiratory virus is selected from the group consisting of Adenovirus, Avian Influenza Virus, Bocavirus, Coronavirus, Cytomegalovirus, Hantavirus, Herpes Simplex Virus, Influenza Virus, Measles, Metapneumovirus, Parainfluenza Virus, Respiratory Syncytial Virus, Rhinovirus, and Varicella-zoster Virus.

9. The pharmaceutical composition of claim 8, wherein the coronavirus is any one selected from the group consisting of 229E, NL63, OC43, HKU1, SARS-CoV, MERS-CoV, SARS-CoV-2, porcine epidemic diarrhea virus (PEDV), (porcine) transmissible gastroenteritis virus (TGEV), canine coronavirus (CCoV), feline coronavirus (FCoV), Miniopterus bat coronavirus 1, Miniopterus bat coronavirus HKU8, Rhinolophus bat coronavirus HKU2, Scotophilus bat coronavirus 512, porcine hemagglutinating encephalomyelitis virus (PHEV), bovine coronavirus (BCoV), equine coronavirus (EqCoV), murine coronavirus (MuCoV), Tylonycteris bat coronavirus HKU4, Pipistrellus bat coronavirus HKU5, Rousettus bat coronavirus HKU9, Avian coronavirus, Beluga whale-coronavirus SW1, Bulbul-coronavirus HKU11, Thrush-coronavirus HKU12, and Munia-coronavirus HKU13.

10. The pharmaceutical composition of claim 1, wherein the self-replication infection indicates that the coronavirus replicates in the respiratory cells of the subject and infects surrounding cells.

11. A post-exposure prophylaxis (PEP) method for protecting a subject from self-replication infection by respiratory viruses after exposed to potential respiratory virus infection, wherein a prophylactically effective amount of interferon-beta is administered to a subject.

12. A method for protecting a subject from self-replication infection by respiratory viruses comprising administering a prophylactically effective amount of interferon-beta to a subject after exposed to potential respiratory virus infection.

13. Use of interferon-beta for preparing an agent for restraining self-replication infection of respiratory viruses in a subject exposed to potential respiratory virus infection.

14. A method for restraining self-replication infection of respiratory viruses in a subject exposed to potential respiratory virus infection, the method comprising administering an effective amount of a composition comprising interferon-beta as an active ingredient to a subject in need thereof.
